(19) Europäisches Patentamt
European Patent Office
Office européen des brevets

(11) Veröffentlichungsnummer: **0 456 040 A1**

(12) **EUROPÄISCHE PATENTANMELDUNG**

(21) Anmeldenummer: **91106654.6**

(22) Anmeldetag: **25.04.91**

(51) Int. Cl.5: **C07C 49/84**, C07D 295/185, C08F 2/50, C09D 167/00

(30) Priorität: **07.05.90 DE 4014489**

(43) Veröffentlichungstag der Anmeldung: **13.11.91 Patentblatt 91/46**

(84) Benannte Vertragsstaaten: **DE FR GB**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Greber, Gerhard, Prof.**
**Anzengruberstrasse 11**
**A-2540 Bad Vöslau(AT)**
Erfinder: **Poetsch, Eike, Dr.**
**Am Buchwald 4**
**W-6109 Mühltal(DE)**
Erfinder: **Ohngemach, Jörg, Dr.**
**Taubenstrasse 6**
**W-6107 Reinhem(DE)**
Erfinder: **Köhler, Manfred, Dr.**
**Kehlerstrasse 15**
**W-7800 Freiburg(DE)**
Erfinder: **Gruber, Heinrich, Dr.**
**Cottagegasse 82**
**A-1190 Wien(AT)**
Erfinder: **Klos, Robert, Dr.**
**Mariannengasse 24/2/20**
**A-1090 Wien(AT)**

(54) **Copolymerisierbare Benzilketal-Fotoinitiatoren.**

(57) Die Erfindung betrifft neue ungesättigte Fotoinitiatoren vom Benzilketal-Typ und ihre Anwendung in strahlungshärtbaren Bindemittelsystemen.

EP 0 456 040 A1

Die Erfindung betrifft neue Fotoinitiatoren vom Benzilketal-Typ. Es handelt sich hierbei um Molekülstrukturen, die in den ketalischen Alkoholgruppen ethylenisch ungesättigte Gruppen vom Acryl- bzw. Allyl-Typ enthalten. Diese Verbindungen dienen als copolymerisierbare Fotoinitiatoren für die Fotopolymerisation ethylenisch ungesättigter Verbindungen oder solche enthaltender Bindemittelsysteme. Sie zeigen hierbei eine Reihe vielfach unerwarteter Vorteile gegenüber bekannten Fotoinitiatoren.

Fotochemisch induzierte Polymerisationsreaktionen haben in der Technik große Bedeutung erlangt, insbesondere wenn es um eine schnelle Härtung von dünnen Schichten geht, wie z.B. bei der Härtung von Lacküberzügen und Kunststoffbeschichtungen auf Papier, Holz, Metall und Kunststoff oder bei der Trocknung von Druckfarben. Dabei zeichnet sich die Strahlungshärtung in Gegenwart von Fotoinitiatoren gegenüber konventionellen Methoden zum Trocknen oder Härten von Beschichtungen durch Rohstoff- und Energieersparnis, geringe thermische Belastung des Untergrundes, und insbesondere eine hohe Geschwindigkeit aus. Aber auch die Herstellung von Polymermaterialien an sich durch Polymerisation entsprechender ungesättigter monomerer Ausgangsmaterialien erfolgt vielfach fotochemisch und mit Hilfe von Fotoinitiatoren, wobei übliche Verfahren, wie Lösungs- und Emulsionspolymerisationen zur Anwendung gelangen.

Da bei den genannten Reaktionen in der Regel keiner der Reaktionspartner in der Lage ist, die fotochemisch wirksame Strahlung in ausreichendem Maße zu absorbieren, müssen Fotoinitiatoren zugesetzt werden, die in der Lage sind, entweder eingestrahlte energiereiche Strahlung, meist UV-Licht, zu absorbieren und dabei aktive Starterradikale zu bilden, die ihrerseits die Fotopolymerisation auslösen, oder die aufgenommene Energie zur Radikalbildung auf einen der polymerisierbaren Reaktionspartner zu übertragen. An der eigentlichen Polymerisationsreaktion nehmen die Initiatoren normalerweise nicht teil.

Hierfür geeignete Fotoinitiatoren üblicher Art sind überwiegend Verbindungen vom Typ der aromatischen Ketone, wie Benzophenon, Benzil oder Thioxanthone sowie vom Typ der $\alpha$-substituierten Alkylphenone, wie Benzoinether, Benzilketale, Dialkoxyacetophenone oder Hydroxyalkylphenone, sowie von diesen Strukturtypen abgeleitete Derivate.

Fotoinitiatoren vom Typ der aromatischen Ketone gehören zur Gruppe der "intermolekularen H-Abstraktoren", die Fotoinitiatoren vom Typ der a-substituierten Alkylphenone gehören zur Gruppe der "intramolekularen $\alpha$-Spalter". Intramolekulare $\alpha$-Spalter zerfallen nach der Strahlungsanregung und Übergang in den angeregten Triplett-Zustand in zwei aktive Starterradikale. Intermolekulare H-Abstraktoren benötigen für eine effektive Wirksamkeit Coinitiatoren, meist werden hierfür Amine eingesetzt, von denen sie im angeregten Triplett-Zustand ein Wasserstoffatom abstrahieren und hierdurch die aktiven Starterradikale bilden.

Die fortschreitende Diversifizierung und Spezialisierung von Verfahren und Produkten auf dem Sektor der Beschichtungstechnik mit Polymermaterialien und das vermehrt einhergehende Erfordernis von maßgeschneiderten Probemlösungen bringt es mit sich, daß auch an Fotoinitiatoren immer höhere und spezifischere Anforderungen gestellt werden. Fotoinitiatoren bekannter Art erfüllen daher vielfach nicht oder zumindest nicht optimal die heute an sie gestellten Forderungen.

Wesentliche Problemkreise sind hierbei die Erzielung einer maximalen Fotoinitiator-Wirkung, vor allem auch bei dicken Beschichtungen sowie in pigmentierten Systemen, die Kompatibilität mit den unterschiedlichsten Bindemittelsystemen, ihren reaktiven Bestandteilen und sonstigen modifizierenden Zusätzen, die Dunkellagerstabilität der mit dem Initiator versehenen Systeme und die möglichen Qualitätsbeeinträchtigungen des gehärteten Endproduktes, wie Vergilbung, durch unumgesetzte Initiatorreste und Initiatorabbauprodukte.

Letzterem Problem wird dadurch begegnet, daß man bekannte Fotoinitiatoren mit ungesättigten Gruppen substituiert. Hierdurch wird ein migrationsfester Einbau durch Copolymerisation von Fotoinitiator- bzw. seiner Fotolyseprodukte in das strahlungsgehärtete Endprodukt bewirkt. In DE 35 34 645 sind beispielsweise copolymerisierbare Fotoinitiatoren vom Hydroxyalkylphenon-Typ und ihre entsprechende Anwendung beschrieben. Der Effekt dieser Modifikation, nämlich der Grad der Einpolymerisation in das gehärtete System, läßt sich durch Extraktionsversuche bestimmen.

Es hat sich allerdings gezeigt, daß diese ungesättigt modifizierten Hydroxyalkylphenon-Fotoinitiatoren nicht mit gleichem Erfolg in beliebigen strahlungshärtbaren Bindemittelsystemen einsetzbar sind. In Bindemittelsystemen auf Basis von in Styrol gelösten ungesättigten Polyesterharzen sogenannten UP-Harz-Systemen, sind Fotoinitiatoren vom Hydroxyalkylphenon-Typ generell weniger geeignet, da mit ihnen unter üblichen Verarbeitungsbedingungen keine ausreichende Härtung zu erzielen ist. Dies trifft insbesondere zu, wenn diese Systeme in dickerer Schicht, etwa mit Schichtdicken zwischen 100 und 500 $\mu$m, angewendet werden sollen. Dies ist in der Praxis bei der Beschichtung von Holzoberflächen für Zwecke des Möbelbaus der Fall. Auch die copolymerisierbaren Fotoinitiatoren von DE 35 34 645 bringen hierfür keinen Vorteil. Geeignete Fotoinitiatoren für UP-Harz-Systeme sind vom Typ der Benzoinether und Benzilketale, wobei Benzildimethylketal hierfür bislang der optimale Initiator ist. Initiatoren dieser Typen werden in das System

jedoch nicht einpolymerisiert, was sich in einem hohen extrahierbaren Anteil zeigt. Die ungesättigten Fotoinitiatoren α-Allylbenzoin und α-Allylbenzoinallylether bewirken in UP-Harz-Systemen ebenfalls nur ungenügende Härtung und ihr extrahierbarer Anteil ist entsprechend groß.

Der Erfindung lag daher die Aufgabe zugrunde, für UP-Harz-Systeme geeignete copolymerisierbare Fotoinitiatoren zu finden, die möglichst eine dem Benzildimethylketal entsprechende Härtung bewirken und die sich migrationsfest in das System einpolymerisieren lassen.

Überraschenderweise wurde nun gefunden, daß Verbindungen der Formel I

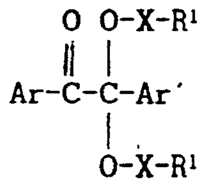 (I)

worin

Ar, Ar' jeweils Aryl mit bis zu 12 C-Atomen gegebenenfalls ein- oder mehrfach substituiert mit $C_{1-6}$-Alkyl oder -Alkoxy,

X eine direkte Bindung, $[(CH_2)_m-O]_n$,

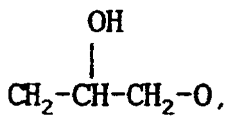, 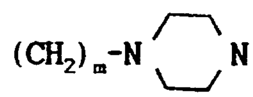

$R^1$

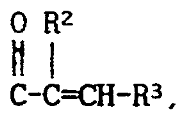, $CH_2-CH=CH-R^3$

$R^2$, $R^3$ H, $C_{1-6}$-Alkyl, Cl, CN, $COOR^4$

$R^4$ H, $C_{1-6}$-Alkyl

n, m die Zahlen 1 bis 6 bedeuten, diese Anforderungen in vorzüglicher Weise erfüllen.

Gegenstand der Erfindung sind Verbindungen der Formel I.

Gegenstand der Erfindung ist weiterhin die Verwendung von Verbindungen der Formel I als Fotoinitiatoren für die Strahlungshärtung ethylenisch ungesättigte Verbindungen enthaltender Bindemittelsysteme.

Gegenstand der Erfindung sind darüber hinaus strahlungshärtbare Bindemittelsysteme auf Basis von in Styrol gelösten ungesättigten Polyesterharzen, die eine wirksame Menge mindestens einer Verbindung der Formel I als Fotoinitiator enthalten.

Gegenstand der Erfindung ist schließlich ein Verfahren zur Herstellung einer strahlungsgehärteten Beschichtung auf einem Substrat, wobei man dieses mit einem derartigen strahlungshärtbaren Bindemittelsystem, das mindestens eine Verbindung der Formel I als Fotoinitiator enthält, beschichtet und die Härtung durch Bestrahlen mit UV-Licht einer Wellenlänge zwischen 250 und 450 nm vornimmt.

Die Verbindungen der Formel I sind neu. Es handelt sich hierbei um Verbindungen vom Typ der Benzilketale, in denen die ketalischen Alkoholgruppen ethylenisch ungesättigte Gruppen aufweisen. In Formel I bedeuten Ar und Ar' jeweils Aryl mit bis zu 12 C-Atomen, die gegebenenfalls ein- oder mehrfach mit Alkyl oder Alkoxy mit 1 bis 6 C-Atomen substituiert sein können. Vorzugsweise bedeuten Ar und Ar' jeweils Phenyl. X kann eine direkte Bindung, eine Alkylenoxygruppe mit 1 bis 6 C-Atomen oder eine aus 2 bis 6 derartigen Gruppen aufgebaute Polyalkylenoxygruppe sein. Weiterhin kann X eine 2-Hydroxypropylenoxygruppe oder eine N-Alkylenpiperazinogruppe, in der die Alkylengruppe 1 bis 6 C-Atomen enthalten kann, bedeuten. $R^1$ kann jeweils eine Acryl- oder Allylgruppe sein, welche wiederum mit $C_{1-6}$-Alkyl, Chlor, Cyano, Hydroxycarbonyl oder $C_{1-6}$-Alkoxycarbonyl substituiert sein können.

Typische erfindungsgemäße Verbindungen sind beispielsweise

Benzil-di-(2-acryloyloxyethyl)-ketal (Ia)
Benzil-di-(2-methacryloyloxyethyl)-ketal (Ib)
Benzil-di-(2-hydroxy-3-allyloxypropyl)-ketal (Ic)
Benzil-di-[2-N-(N'-acryloyl)-piperazinoethyl]-ketal (Id)
Benzil-di-[2-N-(N'-methacryloyl)-piperazinoethyl]-ketal (Ie)

Besonders bevorzugt ist die Verbindung Ie.

Die Herstellung der Verbindungen der Formel I erfolgt mit Hilfe gängiger Reaktionen, wie insbesondere Ketalisierungs-, Veretherungs-, Veresterungs- oder Amidierungsreaktionen, wobei insbesondere Benzil als Ausgangsprodukt dient.

So kann beispielsweise die Verbindung Ie durch Umsetzung von Benzil mit Chlorethanol in Gegenwart von Thionylchlorid, anschließende Umsetzung mit Piperazin und schließlich Umsetzung mit Methacrylsäurechlorid in Gegenwart von Triethylamin erhalten werden.

Die Reaktionsbedingungen hierbei entsprechen dem Üblichen und können den Standardwerken der präparativen organischen Chemie entnommen werden, z.B. HOUBEN-WEYL, Methoden der organischen Chemie, Georg-Thieme Verlag, Stuttgart, oder ORGANIC SYNTHESIS, J. Wiley, New York London Sydney.

Die erfindungsgemäßen Verbindungen der Formel I können sehr vorteilhaft als Fotoinitiatoren für die Fotopolymerisation ethylenisch ungesättigter Verbindungen bzw. solche enthaltender Bindemittelsysteme eingesetzt werden. Vornehmlich dienen sie analog zu bekannten Fotoinitiatoren als UV-Härter für Lack- oder Polymerbeschichtungen sowie Druckfarben auf Basis UV-härtbarer Bindemittel- oder Hybridbindemittelsysteme, die auch in Form wäßriger Dispersionen vorliegen können. Die Anwendung erfolgt in üblicher Weise, wobei sie den Bindemittelsystemen in der Regel in Mengen von 0,1 bis 20 Gew.%, vorzugsweise 1 bis 10 Gew.% zugesetzt werden. Dieser Zusatz geschieht in der Regel durch direktes Einrühren, gegebenenfalls unter Zuhilfenahme einer untergeordneten Menge eines kompatiblen Lösungsmittels. Hierbei zeigen die Verbindungen der Formel I sehr günstige Eigenschaften in Bezug auf ihre Löslichkeit bzw. homogene Mischbarkeit mit praktisch allen üblichen strahlungshärtbaren Systemen sowie in Bezug auf die Dunkellagerstabilität der mit ihnen versetzten Systeme.

Unter einem fotopolymerisierbaren Bindemittelsystem wird üblicherweise ein Gemisch verstanden, das mindestens eine ethylenisch ungesättigte, durch freie Radikale fotopolymerisierbare Verbindung sowie gegebenenfalls weitere übliche Zusatzstoffe enthält. Als radikalisch polymerisierbare Komponenten sind praktisch alle Materialien, die olefinisch ungesättigte Doppelbindungen aufweisen, geeignet. Es können dies insbesondere Monomere, Oligomere und Polymere mit jeweils zumindest einer oder zweckmäßigerweise mit mehreren ungesättigten Funktionen vom Acrylat- oder Vinyl-Typ sein. Entsprechende Materialien sind dem Fachmann in großer Fülle bekannt. Beispiele für solche Verbindungen sind Acrylverbindungen, insbesondere Acrylate von aliphatischen und aromatischen Mono- oder Polyhydroxyverbindungen, wie etwa (Meth)acrylsäure einschließlich deren Salze und Amide, (Meth)acrylnitril, (Meth)acrylsäurealkylester, Hydroxyethyl(meth)acrylat, Vinyl(meth)acrylat, Ethylendi(meth)acrylat, 1,6-Hexandioldi(meth)acrylat, Trimethylolpropandi(meth)acrylat, Pentaerythrittri(meth)acrylat, weiterhin Vinylverbindungen wie etwa Vinylchlorid, Vinylidenchlorid, Styrol, Divinylbenzol, N-Vinylpyrrolidion, N-Vinylcarbazol. Beispiele für höhermolekulare und polymere Bindemittelkomponenten sind etwa acrylierte Polyester-, Acrylat-, Epoxy-, Urethan-, Polyamid- und Siliconharze. Überwiegend sind strahlungshärtbare Bindemittelsysteme Gemische mehrerer der aufgeführten niedermolekularen und höhermolekularen ungesättigten Komponenten.

Hybridbindemittelsysteme enthalten darüber hinaus noch Komponenten, die thermisch, beispielsweise durch Polykondensation oder Polyaddition, nicht jedoch radikalisch vernetzt werden können. Beispiele hierfür sind etwa säurehärtbare Melaminharze sowie Polyurethan- oder Polyester-bildende Reaktivharze.

Die strahlungshärtbaren Bindemittelsysteme können auch in Form wäßriger Dispersionen vorliegen, deren Wasseranteil nach erfolgter Beschichtung normalerweise durch kurzen Erhitzen entfernt wird.

Besonders vorteilhaft ist die Anwendung der erfindungsgemäßen Verbindungen der Formel I als Fotoinitiatoren in Systemen, in denen sich bisher Fotoinitiatoren vom Benzilketal- bzw. Benzoinether-Typ als günstig erwiesen haben. Dies trifft insbesondere auf Systeme zu, die auf in Styrol gelösten ungesättigten Polyesterharzen basieren. Derartige UP-Harz-Systeme, die üblicherweise olefinisch ungesättigte Polyesterharze enthalten, stehen aus industrieller Fertigung reichhaltig zur Verfügung.

Durch den ungesättigten Charakter der erfindungsgemäßen Fotoinitiatoren der Formel I wird ein hoher Grad an kovalentem Einbau von Fotoinitiator bzw. seiner Fotolyseprodukte erreicht. Im Vergleich liegt für erfindungsgemäße Fotoinitiatoren bei optimalen Härtungsbedingungen der aus der gehärteten Beschichtung

extrahierbare Anteil überraschenderweise bei ca. 25 % oder weniger, während für Benzildimethylketal und andere übliche Initiatoren der extrahierbare Anteil 2 bis 3mal so hoch ist.

Ein besonderer Vorteil ist die besonders hohe Reaktivität der erfindungsgemäßen Fotoinitiatoren der Formel I, die vergleichbar oder sogar höher als in den zugrundeliegenden Fotoinitiatortypen ist. Dies trifft insbesondere auch für die Anwendung in Beschichtungen mit hoher Schichtdicke etwa über 100 nm sowie für pigmentierte Systeme zu. So lassen sich mit den Verbindungen der Formel I bei äquivalentem Mengeneinsatz und vergleichbaren Härtungsbedingungen höhere Schichthärten erzielen als mit bekannten Fotoinitiatoren gleicher oder verwandter Grundstruktur. Für eine vorbestimmte Schichthärte ist eine geringere Strahlungsleistung bzw. eine kürze Bestrahlungsdauer erforderlich.

Mit den erfindungsgemäßen Verbindungen der Formel I werden erstmals speziell für die Anwendung in UP-Harz-Systemen besonders geeignete Fotoinitiatoren bereitgestellt, die in sich höchste Härtungsleistung und migrationsfesten kovalenten Einbau durch Copolymerisation mit den Systembestandteilen vereinen.

Die mit den Verbindungen der Formel I als Fotoinitiatoren härtbaren Bindemittelsysteme können in ihren qualitativen wie quantitativen Zusammensetzungen in weiten Bereichen variiert werden und sie können insbesondere auch weitere Bestandteile und Zusätze enthalten. Zweckmäßigerweise sollte hierbei der Anteil an reaktiven Komponenten 10 Gew.% nicht unterschreiten. Als weitere Bestandteile und Zusätze in den für den jeweiligen Zweck angebrachten und üblichen Mengen können anorganische und organische Pigmente, Farbstoffe, Füllstoffe, Verlaufsmittel, oberflächenaktive Mittel, Lichtstabilisatoren und Antioxydantien, Thixotropiemittel, Mattierungsmittel, Weichmacher, Lösungsmittel, Dispergierhilfsmittel, weitere Bindemittel und Harze, sonstige Fotoinitiatoren, spektrale Sensibilisatoren und Coinitiatoren bekannter Art, weitere thermo- bzw. photoreaktive Radikalstarter sowie Kationen- oder säurebildende Katalysatoren enthalten sein.

Die genannten Bindemittelsysteme können mit den Verbindungen der Formel I erfindungsgemäß durch Einwirkung von Strahlungsenergie, insbesondere aus dem UV-Wellenlängenbereich gehärtet werden. In bestimmten Fällen, vornehmlich bei Hybridbindemittelsystemen, ist es zweckmäßig, die Aushärtung durch gleichzeitige oder nachfolgende Zufuhr von Wärmeenergie zu fördern.

Die Fotopolymerisation erfolgt nach an sich bekannten Methoden durch Bestrahlen mit Licht oder UV-Strahlung des Wellenlängenbereichs von 250-450 nm, vorzugsweise von 300-400 nm. Als Strahlenquellen können die üblichen Quecksilberdampf-Hochdruck-,-Mitteldruck- oder -Niederdrucklampen sowie Xenon- und Wolframlampen verwendet werden.

Die Durchführung der Fotopolymerisation unter Verwendung der erfindungsgemäßen Fotoinitiator der Formel I kann sowohl diskontinuierlich als auch kontinuierlich geschehen. Die Bestrahlungsdauer hängt von der Art der Durchführung, von der Art und Menge der eingesetzten polymerisierbaren Materialien, von Art und Konzentration der verwendeten Fotoinitiatoren und von der Intensität der Lichtquelle ab. Bei der Strahlungshärtung von Beschichtungen liegt sie üblicherweise im Bereich weniger Sekunden bis Minuten.

Die erfindungsgemäßen Verbindungen der Formel I werden bevorzugt verwendet als Fotoinitiatoren bei der UV-Härtung von dünnen Schichten wie beispielsweise Lackbeschichtungen auf allen hierfür üblichen Materialien und Trägern. Diese können vornehmlich sein Papier, Holz, textile Träger, Kunststoff, Glas, Keramik und Metall. Ein wichtiges Anwendungsgebiet ist auch die Trocknung bzw. Härtung von Druckfarben und Siebdruckmaterialien, von denen letztere bevorzugt bei der Oberflächenbeschichtung bzw. -gestaltung von beispielsweise Dosen, Tuben und metallenen Verschlußkappen eingesetzt werden.

UP-Harz-Systeme werden bevorzugt eingesetzt bei der Beschichtung von Holzoberflächen, beispielsweise in der Möbelproduktion.

Ihre einfache Zugänglichkeit und ihre vorzüglichen anwendungstechnischen Eigenschaften, vor allem ihre hohe Reaktivität in UP-Harz-Systemen, vorzugsweise wenn diese in dicker Schicht appliziert werden, sowie ihre Copolymerisationsfähigkeit, machen die erfindungsgemäßen Fotoinitiatoren für den praktischen Einsatz besonders wertvoll.

<u>Beispiel 1</u>

Benzil-di-[2-N-(N'-methacryloyl)-piperazinoethyl]-ketal (Ie)

(a) Benzil-di-(2-chlorethyl)-ketal

Eine Suspension von 21 g (0,1 Mol) Benzil und 23,8 g (0,2 Mol) Thionylchlorid wurde auf -5 bis -10 °C abgekühlt und bei dieser Temperatur wurden innerhalb 2 Std. 35,5 g (0,44 Mol) 2-Chlorethanol eingetropft. Man hielt das Reaktionsgemisch weitere 3 Std. bei etwa -5 °C, danach rührte man 10 Std. bei 20 °C und noch weitere 30 min. bei 50 °C. Nach dem Einengen im Vakuum wurde der Rückstand mit 3 g Kaliumcarbonat und 0,6 ml Trimethylphosphit versetzt, kurze Zeit gerührt und anschließend in 40 ml Isopropanol aufgenommen. Durch Zugabe von wenig Wasser entstand ein kristalliner Niederschlag.

Dieser wurde abgesaugt, mit Wasser gewaschen und aus Isopropanol umkristallisiert.
Ausbeute: 20,6 g farblose Kristalle.
Schmelzpunkt: 58-59 °C.
IR-Spektrum (KBr):
$\nu$ = 1695 $cm^{-1}$ (CO),
1082-1026 $cm^{-1}$ (ketalische CO).
(b) Benzil-di-(2-N-piperazinoethyl)-ketal
19,4 g (54,61 mMol) der Verbindung aus (a) wurden unter Stickstoff mit 47,05 g (0,546 Mol) Piperazin versetzt und 2 Std. bei 120 °C gehalten. Nach dem Erkalten wurde das Rohprodukt in Chloroform gelöst und die organische Schicht mit Wasser neutral gewaschen, getrocknet, eingeengt und im Hochvakuum destilliert.
Ausbeute: 21,5 g gelb-rotes Öl.
(c) Zu 24 g (53 mMol) der Verbindung aus (b) in 100 ml abs. Benzol und 32,35 g (0,32 Mol) Triethylamin wurde bei 5 °C 14,4 g (0,16 Mol) Methacrylsäurechlorid in 20 ml Benzol zugetropft und anschließend 40 Std. bei 25 °C gerührt. Das ausgefallene Hydrochlorid wurde abgesaugt und das Filtrat im Vakuum eingeengt. Nach dem Lösen in $CHCl_3$ wurde mit Wasser gewaschen, getrocknet und im Vakuum eingeengt. Das Produkt wurde säulenchromatographisch gereinigt.
Ausbeute: 15 g le als dunkelrotes Öl.
$^1H$-NMR-Spektrum ($CDCl_3$):

| | |
|---|---|
| 7,6-7,1 (m) Aromat. | (10 H) |
| 5,0-4,9 ppm (m) olef. | (4 H) |
| 3,7-3,3 ppm (S) O-$CH_2$ | (4 H) |
| 2,7-2,2 ppm (m) N-$CH_2$ | (20 H) |
| 1,9-1,8 ppm (s) aliph. | (6 H) |

IR-Spektrum (NaCl): $\nu$ = 1695 $cm^{-1}$ (CO)

| Analyse in %: | ber.: | gef.: |
|---|---|---|
| | C = 68,43 | C = 68,23 |
| | H = 7,43 | H = 7,54 |
| | N = 10,70 | N = 11,00 |
| | O = 13,40 | O = 13,19 |

Beispiel 2: Vergleichsversuch

Es wurde die Fotoreaktivität von Initiatoren bei der UV-Härtung eines in dicker Schicht applizierten Bindemittelsystems auf Basis von in Styrol gelöstem UP-Harz bestimmt (als Maß diente die Pendelhärte der gehärteten Schicht).

Weiterhin wurde die Extrahierbarkeit dieser Initiatoren aus der gehärteten Schicht ermittelt.

Versuchsbedingungen

Bindemittelsystem:

100 Gt eines in Styrol gelösten ungesättigten Polyesterharzes (Roskydal 300 der Fa. Bayer)
8 Gt Styrol

Initiierung:

0,01 Mol Initiator

Dicke der applizierten Schicht:

500 µm, auf Glas appliziert mit Filmziehrahmen

Ablüftzeit:

90 Sekunden

UV-Härtung:

| | |
|---|---|
| Labortrockner: | Typ BE 22 der Fa. Beltron |
| Lampenleistung: | 2 UV-Lampen mit jeweils 80 W/cm |
| Härtungsgeschwindigkeit: | 2,5 m/min |
| Belichtungsabstand: | 12 cm |

Pendelhärte:

Messung nach DIN 53 157

Extraktion der UV-gehärteten Schichten

0,20 g der UV-gehärteten Schicht werden zerkleinert und in ein 40-ml-Fläschchen überführt. Dann werden 20 ml Acetonitril p.a. zugegeben. Die anschließende Extraktion wird im Ultraschallbad durchgeführt. Sie dauert 2 Stunden. Nach 15 Stunden wird die überstehende Flüssigkeit abfiltriert und analysiert.

Analyse:

Der extrahierte Initiatorteil wird mittels HPLC (UV-Detektion) bestimmt.

Untersuchungsergebnisse:

Die Ergebnisse zeigt nachfolgende Tabelle 1: Der erfindungsgemäße Initiator zeigt bei höchster Härtungsleistung die geringste Extrahierbarkeit.

Tabelle: Untersuchungsergebnisse

| Initiator | | Pendelhärte | extrahierbarer Anteil |
|---|---|---|---|
| Verbindung I e (erfindungsgemäß) | 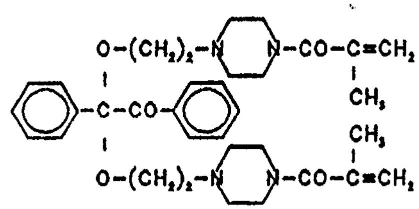 | 116 s | 26,5 % |
| Benzildimethylketal (Vergleich) | 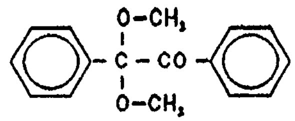 | 107 s | 64,3 % |
| Benzoinisopropylether (Vergleich) | 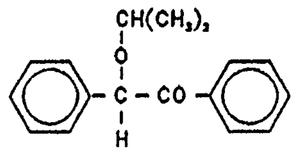 | 102 s | 75,5 % |
| α-Allylbenzoin (Vegleich) | 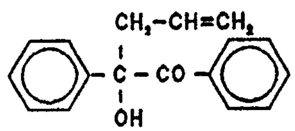 | 31 s | 82,5 % |
| Initiatorgemäß DE 35 34 645 (Vergleich) | 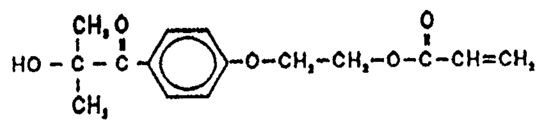 | keine Durchhärtung | -- |

## Patentansprüche

1. Verbindungen der Formel I

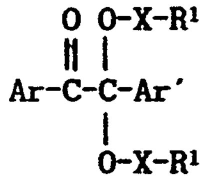

(I)

worin

Ar, Ar' jeweils mit bis zu 12 C-Atomen, gegebenenfalls ein- oder mehrfach substituiert mit $C_{1-6}$-Alkyl oder -Alkoxy,

X eine direkte Bindung, $[(CH_2)_m\text{-}O]_n$,

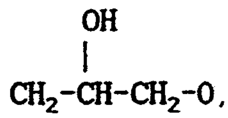, 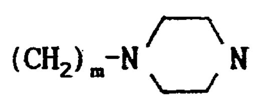

$R^1$

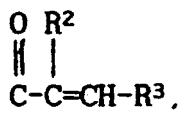, $CH_2\text{-}CH{=}CH\text{-}R^3$

$R^2$, $R^3$ H, $C_{1-6}$-Alkyl, Cl, CN, $COOR^4$

$R^4$ H, $C_{1-6}$-Alkyl

n, m die Zahlen 1 bis 6

bedeuten.

2. Verwendung der Verbindungen der Formel I als Fotoinitiatoren für die Strahlungshärtung ethylenisch ungesättigte Verbindungen enthaltender Bindemittelsysteme.

3. Strahlungshärtbare Bindemittelsysteme auf Basis von in Styrol gelösten ungesättigten Polyesterharzen, dadurch gekennzeichnet, daß sie eine wirksame Menge mindestens eine Verbindung der Formel I als Fotoinitiator enthalten.

4. Strahlungshärtbare Bindemittelsysteme nach Anspruch 3, dadurch gekennzeichnet, daß sie 0, 1 bis 20 Gew.%, vorzugsweise 1 bis 10 Gew.%., einer Verbindung der Formel I enthalten.

5. Verfahren zur Herstellung einer strahlungsgehärteten Beschichtung auf einem Substrat, dadurch gekennzeichent, daß man dieses mit einem Bindemittelsystem nach Anspruch 3 oder 4 beschichtet und die Härtung durch Bestrahlen mit UV-Licht eine Wellenlänge zwischen 250 und 450 nm vornimmt.

Europäisches Patentamt

# EUROPÄISCHER RECHERCHENBERICHT

Nummer der Anmeldung

EP 91106654.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP - A1 - 0 002 707 (CIBA-GEIGY AG) * Zusammenfassung; Patentansprüche 1,8,11,12,16 * | 1,2,3 | C 07 C 49/84<br>C 07 D 295/185<br>C 08 F 2/50<br>C 09 D 167/00 |
| A | EP - A1 - 0 242 330 (CIBA-GEIGY AG) * Zusammenfassung; Patentansprüche 8,12 * | 1,2,3 | |
| D,A | DE - A1 - 3 534 645 (MERCK PATENT GMBH) * Zusammenfassung * | 1,2 | |
| A | AT - B - 331 237 (CIBA-GEIGY AG) * Patentanspruch 1 * | 1 | |
| A | CHEMICAL ABSTRACTS, Band 104, Nr. 24, 16. Juni 1986, Columbus, Ohio, USA K. SUZUKI et al. "Supports for relief printing plate material with photosensitive polymers.." Seite 610, rechte Spalte, Zusammenfassung-Nr. 216 555y & Jpn. Tokkyo Koho JP 60 52,420 (85 52,420) | 1,2,3 | **RECHERCHIERTE SACHGEBIETE (Int. Cl.5)**<br>C 07 C 49/00<br>C 07 D 295/00<br>C 08 F 2/00<br>C 09 D 167/00 |
| A | CHEMICAL ABSTRACTS; Band 94, Nr. 11, 16. März 1981, Columbus, Ohio, USA TEIJIN LTD. "Benzil ketals" Seite 734, linke Spalte, Zusammenfassung-Nr. 84 172j & Jpn. Kokai Tokkyo Koho 80,104,264 | 1 | |
| A | CHEMICAL ABSTRACTS, Band 91, | 1,2,3 | |

Der vorliegende Recherchenbericht wurde für alle Patentansprüche erstellt.

| Recherchenort | Abschlußdatum der Recherche | Prüfer |
|---|---|---|
| WIEN | 14-08-1991 | SCHNASS |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

-2-
EP 91106654.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| | Nr. 4, 23. Juli 1979, Columbus, Ohio, USA M. YAMAURA et al. "Photocurable polyester compositions." Seite 69, linke Spalte, Zusammenfassung-Nr. 22 078n & Jpn. Kokai Tokkyo Koho 79 24,987 ---- | | |

RECHERCHIERTE SACHGEBIETE (Int. Cl.⁵)

Der vorliegende Recherchenbericht wurde für alle Patentansprüche erstellt.

| Recherchenort | Abschlußdatum der Recherche | Prüfer |
|---|---|---|
| WIEN | 14-08-1991 | SCHNASS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82